(19) Europäisches Patentamt

European Patent Office

Office européen des brevets

(11) Publication number: **0 243 145 B1**

## EUROPEAN PATENT SPECIFICATION

(45) Date of publication of patent specification: **14.07.93** (51) Int. Cl.⁵: **A61K 31/20**, A61K 31/23

(21) Application number: **87303487.0**

(22) Date of filing: **21.04.87**

Divisional application 92119054.2 filed on 21/04/87.

(54) **Topical antimicrobial pharmaceutical compositions.**

(30) Priority: **21.04.86 US 854154**

(43) Date of publication of application:
**28.10.87 Bulletin 87/44**

(45) Publication of the grant of the patent:
**14.07.93 Bulletin 93/28**

(84) Designated Contracting States:
**BE CH DE ES FR GB IT LI NL**

(56) References cited:
**EP-A- 0 143 245**
**EP-A- 0 161 425**
**US-A- 4 474 678**

(73) Proprietor: **Kabara, Jon Joseph**
**414 Green Street**
**Galena Illinois 61036(US)**

(72) Inventor: **Kabara, Jon Joseph**
**414 Green Street**
**Galena Illinois 61036(US)**

(74) Representative: **Collier, Jeremy Austin Grey et al**
**J.A.Kemp & Co. 14, South Square Gray's Inn**
**London WC1R 5EU (GB)**

## Description

The present invention relates generally to topical pharmaceutical compositions and, more particularly, to primarily aqueous solutions exhibiting unique antimicrobial properties.

The use of antimicrobial agents plays an important part in current medical therapy. This particularly true in dermatology, where the most effective course of treatment for skin, mucous membranes, hair lesions, or infections frequently includes the use of a topical antimicrobial agent. The present invention includes topical compositions and methods which provide unique performance against a broad spectrum of microbes with excellent speed.

Various carboxylic acids are known to be good antimicrobial agents. However when these acidic solutions are directly applied to an animal's skin, they may eg. interfere with wound healing or cause irritation or inflammation. Also, these solutions have poor solubility properties in water which, in turn, limits the efficacy of aqueous solutions employing these materials.

It is also well recognized that there is presently a need for a mild antimicrobial which can be safely applied to skin and mucous membranes, such as an animal's teats, ears and eyes, that will be substantially harmless to the applied area but which will eliminate, arrest, or substantially reduce the growth of eg. bacteria, fungi or mold. The instant invention meets this need.

The art discloses that several different carboxylic acid are generally useful in the suppression of growths such as fungi, bacteria and molds. United States Patent No. US-A-4,406,884 issued to Fawzi discloses a topical antimicrobial composition in the form of an aqueous gel or lotion. This composition contains $C_5$-$C_{12}$ fatty acids and has a pH no greater than about 5. United States Patent No. US-A-4,343,798 issued to Fawzi, teaches a topical antimicrobial anti-inflammatory composition having a pH less than about 5 and containing $C_5$-$C_{12}$ fatty acids together with a corticosteroid component. United States Patent No. 4,489,097 issued to Stone, teaches the addition of anti-fungal/antibacterial materials to sterile compositions. The antifungal/antibacterial material disclosed is a $C_4$-$C_9$ carboxylate antimicrobial agent having a pH of about 6.0 or below. United States Patent No. US-A-4,410,442 issued to Lucas, et al. teaches solutions for use with hydrophilic soft contact lenses containing $C_5$-$C_{12}$ fatty acids, especially octanoic acid. United States Patent No. 4,392,848 issued to Lucas, et al. teaches a catheter having a liquid reservoir of an antimicrobial agent flowing through the lumen of the catheter. The antimicrobial agent disclosed is a straight-chain carboxylic acid or carboxylic acid salt having a $C_4$-$C_9$ chain. United States Patent No. 4,430,381 issued to Harvey, et al. teaches a process for imparting antimicrobial properties to a material. The antimicrobial being a $C_3$-$C_{12}$ alkane, alkene or alkyne monocarboxylate. United States Patent Nos. US-A-4,343,788 and US-A-4,479,795, both issued to Mustacich, et al. teach medical polymers that provide diffusion for certain carboxylate antimicrobial agents. United States Patent No. US-A-4,002,775 issued to Kabara teaches a food grade microbicidal composition having a monoester with a $C_{12}$ aliphatic fatty acid as its primary microbicide. United States Patent No. US-A-1,772,975 issued to Weiland teaches the use of lactic acid, acetic acid, or combinations thereof, as antiseptics at properly adjusted pH levels. United States Patent No. US-A-2,154,449 issued to Hoffman et al. teaches the use of aliphatic $C_3$-$C_{12}$ carboxylic acids and their salts as mold inhibitors in food compositions. United States Patent No. US-A-2,190,714 issued to Hoffman, et al. teaches the addition of a $C_3$-$C_{12}$ carboxylic acid to inhibit growth food products other than margarine and sourdough bread. United States Patent No. US-A-3,404,987 to Kooistra, et al. teaches an antimicrobial containing edible mineral salt and edible acid preservative substances, particularly propionic acid. United States Patent No. US-A-2,466,663 issued Russ, et al. teaches the use of a topical or intravenous caprylic acid solution to combat mycotic infections or growths. United States Patent No. US-A-2,729,586 issued to Peck teaches a therapeutic composition having at least one salt of a $C_3$-$C_{11}$ monocarboxylic acid and water soluble chlorophyll.

Other materials also disclose the use of fatty acids for the suppression of eg. fungi, bacteria or mold. Kabara, J., Medium-chain Fatty Acids and Esters as Antimicrobial Agents, Cosmetic and Drug Preservation, Pgs. 275-304, 1984, teaches the use of $C_6$-$C_{22}$ saturated and unsaturated fatty acids as antimicrobials. Kabara, J., Toxicological, Bactericidal and Fungicidal Properties of Fatty Acids and Some Derivatives, The Journal of the American Oil Chemists' Society, Vol. 56, No. 11, Pages 760A-767A (1979) teaches the applying of fatty acids to animal skin and eyes. Some fatty acids were found to be skin and eye irritants. Kabara, J., Inhibition of Staphylococlus Aureus In a Model Agar-Meat System By Monolaurin: A Research Note, Journal of Food Safety, Vol. 6, Pgs. 197-201 (1984), teaches the use of monolaurin as a food preservative to combat microorganisms. Kabara, J., Antimicrobial Agents from Fatty Acids, JAOCS Vol. 61, No. 2, Pgs. 397-403 (1984) teaches the use of saturated and unsaturated fatty acids as antimicrobial agents. Kabara, J., GRAS Antimicrobia Agents for Cosmetic Products, Journal of the Society of Cosmetic Chemists, Vol. 31, Pgs. 1-10 (1980), teaches the composition of monolaurin, a phenol, di-tert-butyl anisole, and a chelating agent such as ethylenediaminetetracetic acid to be useful in destroying gram positive and gram negative bacteria. Schemmel, R., Lynch, P., Krohn, K., and Kabara, J., Monolaurin as an Anticaries Agent, teaches the use of glycerol-monolaurin in inhibiting development of smooth surface caries in rats innoculated with Streptococcus mutants. Kabara, Jr., Ohkawa, M., Ikekawa, T., Katori, T., and Mishikawa, Y., Examination on Antitumor, Immunological and Plant-Growth Inhibitory Effects of Monoglycerides of Caprylic, Capric, and Lauric Acids and Related Compounds, Pharmacological Effects of Lipids, Volume II, Pgs. 263-272 (1985) teaches the use of the monoglycerides or caprylic, capric and lauric acids for regulating antitumor, immunological, and plant-growth activity. Li, C., and Kabara, J., Effects of Lauricidin on Fomes Annosus and Phellinus Weirii, AOCS Monograph No. 5, Pgs. 45-47 (1978) teaches the use of monolaurin in combating root rot fungi in coniferous forest. Kenney, D., Cosmetic Formulas Preserved With Food-Grade Chemicals, Cosmetics and Toiletries, Part 1, Vol. 97, Pgs. 71-76 (1982) and Kabara, J. and Wernette, C., Cosmetic Formulas Preserved with Food-Grade Chemicals, Cosmetics and Toiletries, Part II, Vol. 97, Pgs. 77-84 (1982) teaches the use of monoglyceride emulsifier, food-grade phenols and a chelator in the preservation of cosmetics. Kabara, J., A New Preservative System For Food, Journal of Food Safety, Volume 4, Pgs. 13-25 (1982) teaches the use of monolaurin, a food grade phenolic, and a chelator as an antimicrobial for the preservation of food. Branan, A. and Davison, P. Antimicrobials in Foods, Marcel Dekker, New York 1983, Pgs. 109-140 teaches the use of saturated, unsaturated and esters of fatty acids as antimicrobials and the use of these compounds for food preservation. Kabara, J., Fatty Acids and Derivatives as Antimicrobial Agents - A Review, AOCS Monograph No. 5, Pgs. 1-14 (1978) teaches the use of saturated, unsaturated and esters of fatty acids as antimicrobials and the use of these compounds for permeating microorganism cellular membranes for killing the microorganism.

The art also teaches many methods of ethoxylation. Nonionic Surfactants, Schick, M.J., Marcel Dekker, Inc., New York (1966) and Dillan, K., Effects of the Ethylene Oxide Distribution on Nonionic Surfactant Properties, JAOCS, Vol. 62, No. 7, Pgs. 1144-1151 (1985) teach the ethoxylation of primary alcohols to produce nonionic surfactants.

The above discussion clearly reflects the ambiguous state of the art with regard to the suitability and selection of fatty acid-based materials as antimicrobials, especially in the topical or preservative mode. The art disclosed materials vary widely in their efficacy and possess an even wider variety of side effects, particularly when employed in veterinary topical materials under adverse or stressful conditions. (The term glyceryl and glycerol are used interchangeably herein when describing fatty acid esters.)

The present invention relates to preservative pharmaceutical compositions and methods, especially those useful when applied topically and/or those which exhibit good shelf stability. The present invention relates to the discovery that the overall antimicrobial efficacy and pharmaceutical acceptability of certain glycerol fatty acid esters can be dramatically increased by either (1) the addition of certain ether groups, particularly ethoxy and propoxy units; (2) by the use in combination with select fatty acid materials, (3) a combination of (1) and (2). Such modified glycerol fatty acid ester materials retain or exhibit an improved overall spectrum and speed of activity while producing fewer or reduced (in severity) side effects. Such a material is especially useful when compared to the unmodified material in that it produces much less irritation at the site of application.

The present invention further relates to the discovery that the spectrum and speed of activity of both modified and unmodified fatty acid esters can be significantly improved when used in a tertiary mixture or in combination with a mixture of two or more $C_6$-$C_{18}$ fatty acids, preferably $C_6$-$C_{12}$ fatty acids. All these materials are in turn employed in combination with a topical carrier. Such materials provide effective topical antimicrobial activity and are accordingly useful in the treatment and prevention of conditions which are caused, or aggravated by microbial organisms (including viruses) on skin and mucous membranes or are otherwise related to microbes.

Also, the compositions of the present invention exhibit exceptional preservative applications. In addition to preserving the final composition and stabilizing the material to increase the efficacy in cold climate or conditions, the present compositions provide outstanding preservative characteristics when added primarily as a preservative in food stuffs, cosmetic formulations and pharmaceutical compositions (topical; parenteral; in intramuscular; and intravenous). The preservative applications are further discussed in EP-A-0 244 144.

For example, such compositions are useful in veterinary applications as a teat dip, an eye medication and an ear medication. A safe and effective amount of the compositions, described above, to an animal in need of such treatment on the area to be treated one or more times daily.

It is well known that in general the ethoxylation or propoxylation of an antimicrobial agent generally renders that agent biologically inactive; at a minimum the activity is substantially reduced. See Nonionic Surfactants, Martin J. Schick, Marcel Dekker, Inc., New York, New York Chap. 28, Pgs. 958-960.

Unexpectedly, it has been found that the addition of a select number of ethoxy or propoxy units to a glyceryl fatty acid ester results in an antimicrobial agent with good activity and reduced side effects. Further, it has been discovered that the faced narrow range ethoxylates possess better surface-active properties when compared with the broad distribution range adducts. Also, the narrow range ethoxylates seem to act faster and have a better detergent activity than the broad distribution adducts; further, this faster germicidal and detergent activity does not correlate with what is expected of non-ionic ethoxylates. Generally, non-ionic ethoxylates such as TWEEW 80 and SPAN 20 are germicidally inactive. While not intending to be bound by theory, it appears that controlled ethoxylation or propoxylation adds to available hydroxyl radicals by ring cleavage with regeneration of the hydroxyl group. This reaction is an addition reaction without termination. Such ethoxylation is discussed in more detail in Dillan, K., Effects of the Ethylene Oxide Distribution of Nonionic Surfactant Properties, JAOCS, Vol. 62, Pgs. 1144-1151, 1985.

The glyceryl fatty acid ester which is to be ethoxylated or propoxylated for use in the compositions and methods of the present invention is preferably selected from polyhydric alcohols, polyglycerols, sucrose, glucose, sorbitol, propylenediol and glyceryl fatty acid esters having about six to about twenty-one carbon atom. Such preferred compounds include monocaprylin, monocaprin, monolaurin, monomyristin, monopalmitolein, α-monopalmitin, monostearin, monoolein, 1-monolinolein, 1-monolinolenin, and mixtures thereof. Still more preferred are monocaprylin, monocaprin, monolaurin, monomyristin, monopalmitolein, monoolein, monoicosenin and monoerucin and mixtures thereof. The highly preferred compounds include monocaprylin, monocaprin and monolaurin and mixtures thereof. Monolaurin is most highly preferred taking into account cost, availability and activity.

The glyceryl fatty acid esters are ethoxylated or propoxylated by conventional ethoxylating or propoxylating compounds and techniques. The compounds are preferably selected from ethylene oxide, propylene oxide, and mixtures thereof, and similar ringed compounds which provide a material which is effective. Most preferably, the ethoxylation compound is selected from ethylene oxide, propylene oxide and mixtures thereof.

The glyceryl fatty acid esters are ethoxylated or propoxylated under controlled conditions preferably within a narrow range according to conventional methods, such as those in the Dillan article. The glyceryl fatty acid esters are ethoxylated or propoxylated by a suitable amount of ethoxylate or propoxylate compound. In a preferred embodiment, the ethoxylation or propoxylation compound is reacted at a level of 0.25 to 25, more preferably 0.5 to 20 moles, more preferably, 0.5 to 3.0 moles and, still more preferably, 0.5 to 1.0 moles of ethoxylate or propoxylate per mole of glyceryl ester. Thus, the resulting products useful in the compositions and methods of the present invention generally contain at least 1 mole of ethoxylate or propoxylate per four moles of glyceryl ester; in other words there is at least one-quarter, and preferably at least one-half, ether unit or moiety per glyceryl fatty acid ester unit or molecule. This could be further expressed as stating that useful ethoxylated glyceryl fatty acid esters of the present invention are esters wherein at least one-quarter, and preferably at least one-half of the total glyceryl ester has reunited with at least one ethoxy or propoxy moiety.

Generally, the adduct formed by the reaction of the glyceryl fatty acid ester and ethoxylation or propoxylation compound occurs as described in the art. However, it is noted that the reaction products are complex and may be formed by other well known conventional processes in the chemical art. For example, the glycerol portion and fatty acid portion may be ethoxylated or propoxylated separately prior to making the final ester.

The ethoxylation or propoxylation adds at least one-quarter, one-half or more ethoxy or propoxy units to the glyceryl fatty acid ester. Preferably the ethoxylation adds 0.5 to 6, more preferably 0.5 to 3 and, still more preferably, 0.5 to 1 ethoxy or propoxy units per final unit of glyceryl fatty acid ester. This generally corresponds to 0.5 to 6 moles of ethoxy or propoxy compound per mole of glyceryl fatty-acid ester.

The pharmaceutical compositions of the present invention employ a safe and effective amount of ethoxy/methoxy modified glyceryl ester adduct in combination with a suitable pharmaceutically-acceptable topical carrier. Such compositions preferably employ 0.025 to 20%, more preferably, 0.1 to 10% and, still more preferably, 1 to 5%, of the ester by weight of the carrier or final composition.

As discussed above, it has been further observed that a combination of a glyceryl fatty acid ester compound (either ethoxylated/propoxylated and non-ethoxylated/propoxylated) in a mixture with at least one and preferably two or more acids selected from $C_6$-$C_{18}$ fatty acids also demonstrates remarkable efficacy. Also, other polyols such as eg. polyglyceryl, sucrose, glucose, and sorbitol sugar esters have been found to work satisfactorily when substituted for the glyceryl fatty acid ester. The useful glyceryl fatty acid esters include those selected from glyceryl fatty acid esters having six to twenty-one carbon atoms and fatty acids having six to eighteen carbon atoms. The preferred glycerol fatty acid ester compounds include monocaprylin, monocaprin, monolaurin, monomyristin, monopalmitolein, α-monopalmitin, monostearin, mon-

4

oolein, 1-monolinolein, 1-monolinolenin, and mixtures thereof. Still more preferred compounds include monocaprylin, monocaprin, monolaurin, monomyristin, monopalmitolein, monoolein, monoicosenoin, and monoerucin and mixtures thereof. The highly preferred compounds include monocaprylin, monocaprin, and monolaurin and mixtures thereof. These materials may also be modified by the addition of one or more ethoxy/propoxy units as described herein prior to being employed in the combination. The tertiary mixtures useful herein comprise a glycerol fatty acid ester, (which say optionally be ethoxylated/propoxylated as described herein); a first fatty acid compound; and a second fatty acid compound. The preferred first and second fatty acid compounds for use in such tertiary mixtures or combinations are straight chain materials and include, without limitation, caproic, heptanoic, caprylic, pelargonic, capric, undecanoic, lauric, myristic, palmitic, heptadecanoic and stearic. The most preferred materials include carproic, heptanoic, caprylic, capric, undecanoic, and lauric. Highly preferred materials include heptanoic, caprylic and capric.

The glyceryl fatty acid esters, first fatty acid, and second fatty acid are added to a pharmaceutically-acceptable topical carrier in safe and effective amounts. In a preferred embodiment, they are present at a wt:wt ratio of ester: total fatty acid compounds of 1:10 to 10:1; more preferably 1:10 to 1:1, and still more preferably 1:10 to 1:5 with the glyceryl ester being present at a level of 0.5 to 5.0%; more preferably 0.1 to 2.0%; and still more preferably 0.5 to 1.0% of the final composition.

The glyceryl fatty acid esters may be ethoxylated or propoxylated under controlled conditions according to conventional methods, such as described above for use in the combination or mixture.

The compositions of the present invention can be prepared and applied in any suitable form, but are preferably prepared in forms including, without limitation eg. aqueous solutions, lotions, balms, gels, salves, ointments, boluses or suppositories. Accordingly the composition and methods may additionally employ conventional compatible pharmaceutically-acceptable carrier materials useful for such applications. It is desirable that the carrier selected be capable of codissolving or suspending the materials used in the composition. Carrier materials suitable for use in the instant composition, therefore, include those well-known for use in the cosmetic and medical arts as a basis for eg. ointments, lotions, creams, salves, aerosols, suppositories or gels. A preferred carrier generally comprised of alcohols, chelating agents, surfactants, parabens and water.

The alcohols useful in the compositions and methods of the present invention may be selected from the group consisting of propylene glycol, phenoxyethanol, methanol, ethanol, isopropyl alcohol, and mixtures thereof. In a preferred embodiment they are selected from the group propylene glycol, phenoxyethanol, and mixtures thereof; still more preferred is a mixture of propylene glycol and phenoxyethanol.

Surfactants useful in the compositions and methods of the present invention include those selected from the group consisting of sarcosinates, pluronic F68, sodium lauryl sulfate, sorbitan monolaurate, lauryldimethylamineoxide, lauric-diethanolamide, PEG-Esters (polyethylene glycol-dilaurate), coconut hydroxyethyl imidazoline, sodium sulfosuccinate ester of lauric MEA, sodium sulfosuccinate ester of ethoxylated lauryl alcohol, lauric-monoethanolamide, bis-(2-hydroxyethyl) cocoamine oxide•IPA, bis-(2-hydroxyethyl) tallowamine oxide•IPA, dimethylcocoamine oxide•IPA, dimethylcocoamine oxide, (negative inhibitors), polyoxypropylene bases, coconut fatty acid, 2-sulfo-ester, sodium salt, N-coconut oil acyl-N-methyl taurine, sodium salt, lauroyl sarcosine, 30% sodium lauryl sarcosinate, sodium lauroyl sarcosinate, myristoyl sarcosine, oleoyl sarcosine, stearoyl sarcosine, polyoxethelene 21 stearyl ether (0.1% BHA & 0.005% citric acid as preservatives), lauroamphoglycinate, lauroamphocarboxyglycinate, lauroamphocarboxypropinate, lauroamphocarboxyglycinate-sulfanate, sodium lauryl sulfate (66% lauryl, 27% myristyl, 71% cetyl), polyoxyethylene sorbitan mono-oleate, and mixtures thereof. In a preferred embodiment they are sarcosinates, amine oxides, pluronic F68, sodium lauryl sulfate, and mixtures thereof, more preferred are pluronic F68, sodium lauryl sulfate, amine oxides, and mixtures thereof.

Chelating agents useful in the compositions and methods of the present invention include those selected from EDTA, EDTA $(Na)_2$, EDTA $(Na)_4$, TEA, lactic acid, citric acid and mixtures thereof. In a preferred embodiment, they are lactic acid, citric acid, EDTA$(Na)_2$, EDTA $(Na)_4$, TEA, and mixtures thereof, more preferred are lactic acid, EDTA$(Na)_2$, EDTA$(Na)_4$, citric acid and mixtures thereof, highly preferred are lactic acid, EDTA$(Na)_2$, EDTA$(Na)_4$, and mixtures thereof.

Parabens useful in the compositions and methods of the present invention may be selected from methyl and propyl parabens. These may possess the methyl alone or in combination with 1 propyl paraben; or 4 methyl 1 propyl; and mixtures thereof (and ester).

Water q.s. may form the remainder of the carrier and is selected from the group consisting of sterile water, distilled water, deionized water, tap and well water. In a preferred embodiment, they are sterile water, distilled water or deionized water. Emusions may also be employed.

The alcohols discussed above may be employed in the compositions and methods of the present invention at any suitable level. In a preferred embodiment, they are present at a level of 5 to 60%, more

5

preferred at 10 to 30% and, in a highly preferred embodiment, at 20 to 25%, by weight per volume of solution.

The surfactants discussed above may be employed in the compositions and methods of the present invention at any suitable level. In a preferred embodiment, they are present at a level of 0.25 to 10%, more preferred at 2 to 10% and, highly preferred, at 4 to 8%, weight per volume of solution.

The parabens discussed above may be employed in the compositions and methods of the present invention at any suitable level. In a preferred embodiment, they are present at a level of 0.05 to 0.5%, more preferred, at 0.1 to 0.5% and, highly preferred, at 0.1 to 0.3%, weight per volume of solution.

The chelators discussed above may be employed in the compositions of the present invention at any suitable level. In a preferred embodiment, they are present at a level of 0.05 to 3%, more preferred at 0.1 to 0.5%, and, highly preferred, at 0.1 to 0.2%, weight per volume of solution.

The compositions of the present invention may additionally employ adjunct components conventionally found in pharmaceutical compositions in their art-established fashion and at their art-established levels. Thus, for example, the compositions may contain additional compatible pharmaceutically active materials for combination therapy (such as supplementary antimicrobial, antipruritics, astringents, local anaesthetics, or anti-inflammatory agents), or may contain materials useful in physically formulating various dosage forms of the present invention, such as excipients, dyes, perfumes, thickening agents, stabilizers, skin penetration enhancers, preservatives, or antioxidants.

Topical treatment regimens according to the practice of this invention comprise applying a safe and effective amount of the compositions described herein directly to the infected or at-risk skin or mucous membrane; particularly, in a veterinary use, on the teats, eyes, ear areas or at any other situs particularly susceptible to microbial contamination. The solution may be eg. sprayed, dipped, wiped, dropped, poured, toweled, onto the area to be treated. Application can be made once, or preferably several times daily, to prevent eg. bacteria, fungi or mold from forming on the animal's skin, teats, ears and eyes.

The following examples illustrate the invention

## EXAMPLE 1

The following formulae have been found to be active against a substantial group of organisms. Also, these formulae have been found to be substantially non-irritating as an ear/eye preparation.

| | |
|---|---|
| Ethoxylated Glycerol Monolaurin (Monolaurin EO) | 0.05 – 1.0% |
| Caprylic Acid/Capric Acid Mixture | 0.1 – 5.0% |
| Parabens | 0.0 – 0.3% |
| Pluronic F68 | 1.0 – 10% |
| Phenoxylethanol | 0.0 – 1.0% |
| EDTA (Na)$_2$ | 0.05 – 0.3% |
| Water | 82.4 – 98.65% |

Glycerol monolaurin is ethoxylated by about one mole ethylene oxide per mole of glycerol monolaurin by conventional ethoxylation methods as described herein.

## EXAMPLE 2

The following concentrate formula has been found to be active against a selective group of organisms, especially bovine mastitis. Also, the concentrate formula has been found to be non-irritating when applied as a teat dip.

6

| | |
|---|---|
| Ethoxylated Glycerol Monolaurin | 0.5 – 2.0% |
| Caprylic and Capric Acid Mixture | 0.5 – 8.0% |
| Phenoxyethanol | 0.0 – 2.5% |
| Propylene Glycol | 10 –30% |
| Parabens | 0–.0 – 1.0% |
| Pluronic F68 | 5 – 13.0% |
| EDTA (Na)$_2$/Lactic Acid | 1 – 15% |
| H$_2$O | 30 – 80% Monolaurin |

is ethoxylated by about 0.5 to about 1.5 mole of ethylene oxide by conventional methods.

Also the above formulas (Examples I and II) may be used in human or veterinary protocols, e.g., in the treatment of nasal tissue disease, opthalmic disease, fungal corneal infection of horses, pulmonary disease, genital infections, and otitis externa. The formulas may be used in insecticidal or germicidal formulation on human or animal skin and plants, catheters, in egg washing, diapers, and wood preservatives. The formulas are also effective in combating fowl mites, ear mites, and ticks.

The above formulas are effective against yeasts, gram negative and gram position organisms and protozoan, more particularly: C. albicans, C. parapsilosis, S. cerevisiae, E. coli, Ps. aeruginosu, S. epidermis, S. aureus, Bacillus subtilis, Streptococcus faecalis, Streptacoccus pyogenec, Corynebacterium, Strep mutans, and Trichomonus vaginalis.

EXAMPLE 3

The following formula concentrate was tested against a yeast, a gram negative and a gram positive organism:

| | % Weight |
|---|---|
| Ethoxylated Glycerol Monolaurin (Monolaurin EO) | 1.0 |
| Caprylic and Capric Mixture | 1.5 |
| Propylene Glycol | 22 |
| Parabens | 0.5 |
| Phenoxyethanol | 2.5 |
| Pluronic F-68 | 5.0 |
| EDTA (NA)$_2$ | 2.0 |
| dH$_2$O | q.s. |

After the formula was diluted 1:10 in water, the dilute was introduced to E. coli and to C. parapsilosis organisms. The results are as follows:

## E. COLI

| Time | Colony Forming Units/ml |
|---|---|
| $T_O$ | $4.3 \times 10^6$ |
| T=2 mins | <30 |
| T=10 mins | <30 |

## C. PARAPSILOSIS

| Time | Colony Forming Units/ml |
|---|---|
| $T_O$ | $1.7 \times 10^6$ |
| T=2 min. | $2.5 \times 10^3$ |
| T=10 min. | <30 |

The formula of Example III was diluted 1:20 in water and then the dilute was introduced to S. aureus. The results are as follows:

## S. AUREUS

| Time | Colony Forming Units/ml |
|---|---|
| $T_O$ | $1.6 \times 10^6$ |
| T=2 min. | $<5.0 \times 10^1$ |
| T=10 min. | <30 |

The above formula of Example III was diluted 1:8 with water and prophylactically applied to a cow's teats twice a day. As a result, upon analyzing the cow's teats, no substantial growth of micro-organisms was found on the teats. The same treatment was conducted on a cow's teats when the ambient air temperature was substantially below 4.4°C (40°F), and substantially similar results were found. It will be appreciated that substantial growth would normally be found.

EXAMPLE 4

EXAMPLE 4

Formula 4-1

|  | % Weight |
|---|---|
| Monolaurin | 1.0 |
| ( Monolaurin EO) Ethoxylated Glycerol Monolayrin | 1.75 |
| Caprylic and Capric Mixture | 5.0 |
| Pluronic F-68 | 13.0 |
| EDTA (NA)$_2$ | 4.0 |
| H$_2$O | 75.25 |

Formula 4-2

|  | % Weight |
|---|---|
| (Monolaurin EO) Ethoxylated Glycerol Monolayrin | 1.75 |
| Caprylic and Capric Mixture | 6.0 |
| Pluronic F-68 | 13.0 |
| Lactic Acid (85%) | 15.0 |
| H$_2$O | 64.25 |

After the formulas of Example 4-1 and 4-2 were diluted 1:8 in water the dilute was introduced to Strep Agalactial and Staph Aureus organisms. The results illustrate the log reduction of organisms after two minutes of exposure with the dilute. Also listed are the log reduction results of other solutions used in the field.

Log Reduction Of Organisms

|  | Time | Strep Agalactial | Staph Aureus |
|---|---|---|---|
| Example 4-1 | T=2 min. | 3.1 | 2.7 |
| Example 4-2 | T=2 min. | 4.3 | 5.1 |
| Tegragon (Quaternary complex) | T=2 min. | 1.0 | 2.0 |
| Teat Care (Chlorhexadine) | T=2 min. | 1.7 | 2.0 |
| All Day (Sorbic Acid) | T=2 min. | - | 1.8 |
| Quartermate (2000 Iodophor) | T=2 min. | 2.2 | 2.4 |

9

EXAMPLE 5

The following formula was tested against the identified organisms with the following results.

Formula 5

|  | % Weight |
|---|---|
| (Monolaurin EO) Ethoxylated Glycerol Monolayrin | 0.15 |
| Caprylic and Capric Mixture | 0.30 |
| Parabens | 0.05 |
| Dowanol | 0.1 |
| Pluronic F-68 | 5.0 |
| EDTA (Na)$_2$ | 0.1 |
| EDTA (Na)$_4$ | 0.1 |
| d H$_2$O | 94.2 |

Ps AERUGINOSA

| Time | Colony Forming Units/ml |
|---|---|
| $T_o$ | $1.2 \times 10^6$ |
| T=2 min. | <30 |
| T=10 min. | <30 |

E. COLI

| Time | Colony Forming Units/ml |
|---|---|
| $T_o$ | $3.0 \times 10^6$ |
| T=2 min. | <30 |
| T=10 min. | <30 |

10

EXAMPLE 6

The following formula was tested against the identified organism with the following results.

|  | % Weight |
|---|---|
| (Monolaurin EO) Ethoxylated Glycerol Monolaurin | 0.15 |
| Caprylic and Capric Mixture | 0.30 |
| Dowanol | 0.10 |
| Pluronic F-68 | 5.0 |
| EDTA (Na)$_2$ | 0.1 |
| EDTA (Na)$_4$ | 0.1 |
| d H$_2$O | 94.25 |

Ps AERUGINOSA

| Time | Colony Forming Units/ml |
|---|---|
| T$_0$ | $1.2 \times 10^6$ |
| T$_2$ | <30 |
| T$_{10}$ | <30 |

E. COLI

| Time | Colony Forming Units/ml |
|---|---|
| T$_0$ | $3.0 \times 10^6$ |
| T=2 min. | <30 |
| T=10 min. | <30 |

Claims

Claims for the following Contracting States : BE, CH, DE, FR, GB, IT, LI, NL

1. A topical antimicrobial pharmaceutical composition comprising:
    (a) an ethoxylated or propoxylated glyceryl fatty acid ester, and
    (b) a pharmaceutically-acceptable carrier.

2. A topical antimicrobial pharmaceutical composition according to claim 1 comprising:
    (a) an ethoxylated or propoxylated glyceryl fatty acid ester;
    (b) a fatty acid mixture comprising
        (i) a first fatty acid antimicrobial agent which is a $C_6$-$C_{18}$ fatty acid; and
        (ii) a second fatty acid antimicrobial agent which is a $C_6$-$C_{18}$ fatty acid wherein the second fatty acid is not the same as the first fatty acid; and
    (c) a pharmaceutically-acceptable carrier.

11

3. A topical antimicrobial pharmaceutical composition according to claim 2 wherein the ratio by weight of the ester to the first and second fatty acids combined is 1:10 to 10:1.

4. A topical antimicrobial pharmaceutical composition according to claim 2 or 3 wherein the first fatty acid and/or the second fatty acid is a straight chain fatty acid having from six to twelve carbon atoms.

5. A topical antimicrobial pharmaceutical composition according to any one of claims 1 to 4 wherein the glyceryl ester comprises at least 1 mole of ethoxy or propoxy moiety per 4 moles of glyceryl ester.

6. A topical antimicrobial pharmaceutical composition according to claim 5 wherein 0.5 to 5 moles of ethoxy or propoxy moiety are present per mole of glyceryl ester.

7. A topical antimicrobial pharmaceutical composition according to any one of claims 1 to 6 wherein the glyceryl fatty acid ester is a monoester.

8. A topical antimicrobial pharmaceutical composition according to claim 7 wherein the monoester contains 6 to 21 carbon atoms.

9. A topical antimicrobial pharmaceutical composition according to claim 8 wherein the monoester is monocaprylin, monocaprin, monolaurin, monomyristin, monopalmitolein, monolein, monoicosenoin, monoerucin or a mixture thereof.

10. A topical antimicrobial pharmaceutical composition according to any one of claims 7 to 9 wherein the monoester is present in an amount of 0.025 to 20.0% by weight of the composition.

11. A topical antimicrobial pharmaceutical composition according to any one of claims 1 to 10 in which the carrier comprises an alcohol, chelating agent, surfactant, paraben, water, or a mixture thereof.

12. A topical antimicrobial pharmaceutical composition according to claim 11 wherein the pharmaceutical carrier comprises an alcohol which is propylene glycol, phenoxyethanol, methanol, ethanol, isopropyl alcohol, or a mixture thereof; a chelating agent which is EDTA, $Na_2(EDTA)$, $Na_4(EDTA)$, TEA, lactic acid, citric acid or a mixture thereof; a surfactant which is a sarcosinate, an amine oxide, pluronic 68, sodium lauryl sulfate or a mixture thereof; or a paraben which is methyl paraben, propyl paraben or a mixture thereof.

13. A topical antimicrobial pharamaceutical composition according to claim 11 or 12 which comprises 5 percent to 60 percent by volume of the alcohol; 0.25 percent to 10 percent by weight of the surfactant; 0.05 percent to 5 percent by weight of the paraben; 0.05 percent to 4 percent by weight of the chelating agent.

14. A composition according to any one of claims 1 to 13 for use as a topical antimicrobial agent.

15. Use of a composition according to any one of claims 1 to 14 in the preparation of a topical antimicrobial medicament.

**Claims for the following Contracting State : ES**

1. A process for the preparation of a topical antimicrobial pharmaceutical composition which comprises mixing together:
   (a) an ethoxylated or propoxylated glyceryl fatty acid ester; and
   (b) a pharmaceutically acceptable carrier.

2. A process for the preparation of a topical antimicrobial pharmaceutical composition according to claim 1 which comprises mixing together:
   (a) an ethoxylated or propoxylated glyceryl fatty acid ester;
   (b) a fatty acid mixture comprising:
      (i) a first fatty acid antimicrobial agent which is a $C_6$-$C_{18}$ fatty acid; and

(ii) a second fatty acid antimicrobial agent which is a $C_6$-$C_{18}$ fatty acid wherein the second fatty acid is not the same as the first fatty acid; and

(c) a pharmaceutically-acceptable carrier.

3.  A process according to claim 2 wherein the ratio by weight of the ester to the first and second fatty acids combined is 1:10 to 10:1.

4.  A process according to claim 2 or 3 wherein the first fatty acid and/or the second fatty acid is a straight chain fatty acid having from six to twelve carbon atoms.

5.  A process according to any one of claims 1 to 4 in which the glyceryl ester comprises at least 1 mole of ethoxy or propoxy moiety per 4 moles of glyceryl ester.

6.  A process according to claim 5 wherein 0.5 to 5 moles of ethoxy or propoxy moiety are present per mole of glyceryl ester.

7.  A process according to any one of claims 1 to 6 wherein the glyceryl fatty acid ester is a monoester.

8.  A process according to claim 7 wherein the monoester contains 6 to 21 carbon atoms.

9.  A process according to claim 8 wherein the monoester is monocaprylin, monocaprin, monolaurin, monomyristin, monopalmitolein, monolein, monoicosenoin, monoerucin or a mixture thereof.

10. A process according to any one of claims 7 to 9 wherein the monoester is present in an amount of 0.025 to 20.0% by weight of the composition.

11. A process according to any one of claims 1 to 10 in which the carrier used comprises an alcohol, chelating agent, surfactant, paraben, water, or a mixture thereof.

12. A process according to claim 11 wherein the pharmaceutical carrier comprises an alcohol which is propylene glycol, phenoxyethanol, methanol, ethanol, isopropyl alcohol, or a mixture thereof; a chelating agent which is EDTA, $Na_2$(EDTA), $Na_4$(EDTA), TEA, lactic acid, citric acid or a mixture thereof; a surfactant which is a sarcosinate, an amine oxide, pluronic 68, sodium lauryl sulfate or a mixture thereof; or a paraben which is methyl paraben, propyl paraben or a mixture thereof.

13. A process according to claim 11 or 12 wherein the alcohol is used in an amount of 5 percent to 60 percent by volume; the surfactant is used in an amount of 0.25 percent to 10 percent by weight; the paraben is used in an amount of 0.05 percent to 5 percent by weight; the chelating agent is used in an amount of 0.05 percent to 4 percent by weight.

14. A composition as defined in any one of claims 1 to 13 for use as a topical antimicrobial agent.

15. Use of a composition as defined in any one of claims 1 to 13 in the preparation of a topical antimicrobial medicament.

**Patentansprüche**

**Patentansprüche für folgende Vertragsstaaten : BE, CH, DE, FR, GB, IT, LI, NL**

1.  Topische antimikrobielle pharmazeutische Zusammensetzung umfassend:

    (a) einen ethoxylierten oder propoxylierten Glycerylfettsäureester, und

    (b) einen pharmazeutisch verträglichen Träger.

2.  Topische antimikrobielle pharmazeutische Zusammensetzung nach Anspruch 1 umfassend:

    (a) einen ethoxylierten oder propoxylierten Glycerylfettsäureester,

    (b) eine Fettsäuremischung umfassend

    (i) ein erstes antimikrobielles Mittel auf der Grundlage einer Fettsäure, die eine $C_6$-$C_{18}$-Fettsäure ist, und

(ii) ein zweites antimikrobielles Mittel auf der Grundlage einer Fettsäure, die eine $C_6$-$C_{18}$-Fettsäure ist, wobei die zweite Fettsäure der Mischung nicht dieselbe ist wie die erste Fettsäure, und

(c) einen pharmazeutisch verträglichen Träger.

3. Topische antimikrobielle pharmazeutische Zusammensetzung nach Anspruch 2, bei der das Gewichtsverhältnis der Ester zu den kombinierten ersten und zweiten Fettsäuren 1:10 bis 10:1 beträgt.

4. Topische antimikrobielle pharmazeutische Zusammensetzung nach Anspruch 2 oder 3, bei der die erste Fettsäure und/oder die zweite Fettsäure eine geradkettige Fettsäure mit 6 bis 12 Kohlenstoffatomen ist (sind).

5. Topische antimikrobielle pharmazeutische Zusammensetzung nach einem der Ansprüche 1 bis 4, bei der der Glycerylester mindestens 1 Mol Ethoxy- oder Propoxykomponente pro 4 Mol Glycerylester umfaßt.

6. Topische antimikrobielle pharmazeutische Zusammensetzung nach Anspruch 5, bei der 0,5 bis 5 Mol Ethoxy oder Propoxykomponente pro Mol Glycerylester vorhanden sind.

7. Topische antimikrobielle pharmazeutische Zusammensetzung nach einem der Ansprüche 1 bis 6, bei der der Glycerylfettsäureester ein Monoester ist.

8. Topische antimikrobielle pharmazeutische Zusammensetzung nach Anspruch 7, bei der der Monoester 6 bis 21 Kohlenstoffatome enthält.

9. Topische antimikrobielle pharmazeutische Zusammensetzung nach Anspruch 8, bei der der Monoester Monocaprylin, Monocaprin, Monolaurin, Monomyristin, Monopalmitolein, Monoolein, Monoicosenoin, Monoerucin oder eine Mischung davon ist.

10. Topische antimikrobielle pharmazeutische Zusammensetzung nach einem der Ansprüche 7 bis 9, bei der der Monoester in einer Menge von 0,025 bis 20,0 % des Gewichts der Zusammensetzung vorhanden ist.

11. Topische antimikrobielle pharmazeutische Zusammensetzung nach einem der Ansprüche 1 bis 10, bei der der Träger einen Alkohol, einen Chelatbildner, ein oberflächenaktives Mittel, Paraben, Wasser oder eine Mischung davon umfaßt.

12. Topische antimikrobielle pharmazeutische Zusammensetzung nach Anspruch 11, bei der der pharmazeutische Träger einen Alkohol, welcher Propylenglykol, Phenoxyethanol, Methanol, Ethanol, Isopropylalkohol oder eine Mischung davon ist, einen Chelatbildner, welcher EDTA, $Na_2$(EDTA), $Na_4$(EDTA), TEA, Milchsäure, Zitronensäure oder eine Mischung davon ist, ein oberflächenaktives Mittel, welches Sarcosinat, ein Aminoxid, Pluronic 68, Natriumlaurylsulfat oder eine Mischung davon ist, oder ein Paraben umfaßt, welches Methylparaben, Propylparaben oder eine Mischung davon ist.

13. Topische antimikrobielle pharmazeutische Zusammensetzung nach Anspruch 11 oder 12 umfassend 5 bis 60 Vol.-% des Alkohols, 0,25 bis 10 Gew.-% des oberflächenaktiven Mittels, 0,05 bis 5 Gew.-% des Parabens und 0,05 bis 4 Gew.-% des Chelatbildners.

14. Zusammensetzung nach einem der Ansprüche 1 bis 13 zur Verwendung als topisches antimikrobielles Mittel.

15. Verwendung einer Zusammensetzung gemäß einem der Ansprüche 1 bis 14 zur Herstellung eines topischen antimikrobiellen Medikaments.

**Patentansprüche für folgenden Vertragsstaat : ES**

1. Verfahren zur Herstellung einer topischen antimikrobiellen pharmazeutischen Zusammensetzung umfassend das Vermischen von:

(a) einem ethoxylierten oder propoxylierten Glycerylfettsäureester mit

(b) einem pharmazeutisch verträglichen Träger.

2.  Verfahren zur Herstellung einer topischen antimikrobiellen pharmazeutischen Zusammensetzung nach Anspruch 1 umfassend das Vermischen von:

(a) einem ethoxylierten oder propoxylierten Glycerylfettsäureester,

(b) einer Fettsäuremischung umfassend

(i) ein erstes antimikrobielles Mittel auf der Grundlage einer Fettsäure, die eine $C_6$-$C_{18}$-Fettsäure ist, und

(ii) ein zweites antimikrobielles Mittel auf der Grundlage einer Fettsäure, die eine $C_6$-$C_{18}$-Fettsäure ist, wobei die zweite Fettsäure der Mischung nicht dieselbe ist wie die erste Fettsäure, und

(c) einem pharmazeutisch verträglichen Träger.

3.  Verfahren nach Anspruch 2, bei dem das Gewichtsverhältnis der Ester zu den kombinierten ersten und zweiten Fettsäuren 1:10 bis 10:1 beträgt.

4.  Verfahren nach Anspruch 2 oder 3, bei dem die erste Fettsäure und/oder die zweite Fettsäure eine geradkettige Fettsäure mit 6 bis 12 Kohlenstoffatomen ist (sind).

5.  Verfahren nach einem der Ansprüche 1 bis 4, bei dem der Glycerylester mindestens 1 Mol Ethoxy- oder Propoxykomponente pro 4 Mol Glycerylester umfaßt.

6.  Verfahren nach Anspruch 5, bei dem 0,5 bis 5 Mol Ethoxy- oder Propoxykomponente pro Mol Glycerylester vorhanden sind.

7.  Verfahren nach einem der Ansprüche 1 bis 6, bei dem der Glycerylfettsäureester ein Monoester ist.

8.  Verfahren nach Anspruch 7, bei dem der Monoester 6 bis 21 Kohlenstoffatome enthält.

9.  Verfahren nach Anspruch 8, bei dem der Monoester Monocaprylin, Monocaprin, Monolaurin, Monomyristin, Monopalmitolein, Monoolein, Monoicosenoin, Monoerucin oder eine Mischung davon ist.

10. Verfahren nach einem der Ansprüche 7 bis 9, bei dem der Monoester in einer Menge von 0,025 bis 20 % des Gewichts der Zusammensetzung vorhanden ist.

11. Verfahren nach einem der Ansprüche 1 bis 10, bei dem der verwendete Träger einen Alkohol, einen Chelatbildner, ein oberflächenaktives Mittel, Paraben, Wasser oder eine Mischung davon umfaßt.

12. Verfahren nach Anspruch 11, bei dem der pharmazeutische Träger einen Alkohol, welcher Propylenglykol, Phenoxyethanol, Methanol, Ethanol, Isopropylalkohol oder eine Mischung davon ist, einen Chelatbildner, welcher EDTA, $Na_2$(EDTA), $Na_4$(EDTA), TEA, Milchsäure, Zitronensäure oder eine Mischung davon ist, ein oberflächenaktives Mittel, welches Sarcosinat, ein Aminoxid, Pluronic 68, Natriumlaurylsulfat oder eine Mischung davon ist, oder ein Paraben umfaßt, welches Methylparaben, Propylparaben oder eine Mischung davon ist.

13. Verfahren nach Anspruch 11 oder 12, bei dem der Alkohol in einer Menge von 5 bis 60 Vol.-%, das oberflächenaktive Mittel in einer Menge von 0,25 bis 10 Gew.-%, das Paraben in einer Menge von 0,05 bis 5 Gew.-% und der Chelatbildner in einer Menge von 0,05 bis 4 Gew.-% eingesetzt werden.

14. Zusammensetzung gemäß Definition in einem der Ansprüche 1 bis 13 zur Verwendung als topisches antimikrobielles Mittel.

15. Verwendung einer in einem der Ansprüche 1 bis 13 definierten Zusammensetzung bei der Herstellung eines topischen antimikrobiellen Medikaments.

15

EP 0 243 145 B1

**Revendications**
**Revendications pour les Etats contractants suivants : BE, CH, DE, FR, GB, IT, LI, NL**

1. Composition pharmaceutique anti-microbienne topique, comprenant :
   (a) un ester d'acide gras du glycérol, éthoxylé ou propoxylé, et
   (b) un support pharmaceutiquement acceptable.

2. Composition pharmaceutique anti-microbienne topique, selon la revendication 1, comprenant :
   (a) un ester d'acide gras du glycérol, éthoxylé ou propoxylé,
   (b) un mélange d'acides gras comprenant :
      (i) un premier acide gras, agent anti-microbien, qui est un acide gras en $C_6$ à $C_{18}$, et
      (ii) un second acide gras, agent anti-microbien, qui est un acide gras en $C_6$ à $C_{18}$, le second acide gras étant différent du premier acide gras, et
   (c) un support pharmaceutiquement acceptable.

3. Composition pharmaceutique anti-microbienne topique, selon la revendication 2, dans laquelle le rapport en poids de l'ester au premier acide gras et au second acide gras combinés est de 1:10 à 10:1.

4. Composition pharmaceutique anti-microbienne topique, selon la revendication 2 ou 3, dans laquelle le premier acide gras et/ou le second acide gras est un acide gras à chaîne droite, ayant de 6 à 12 atomes de carbone.

5. Composition pharmaceutique anti-microbienne topique, selon l'une quelconque des revendications 1 à 4, dans laquelle l'ester du glycérol comprend au moins 1 mole de fragment éthoxy ou propoxy pour 4 moles d'ester de glycérol.

6. Composition pharmaceutique anti-microbienne topique, selon la revendication 5, dans laquelle 0,5 à 5 moles de fragment éthoxy ou propoxy sont présentes par mole d'ester de glycérol.

7. Composition pharmaceutique anti-microbienne topique, selon l'une quelconque des revendications 1 à 6, dans laquelle l'ester d'acide gras du glycérol est un monoester.

8. Composition pharmaceutique anti-microbienne topique, selon la revendication 7, dans laquelle le monoester contient 6 à 21 atomes de carbone.

9. Composition pharmaceutique anti-microbienne topique, selon la revendication 8, dans laquelle le monoester est la monocapryline, la monocaprine, la monolaurine, la monomyristine, la monopalmitoléine, la monoléine, la monoicosénoïne, la monoérucine ou un mélange de ces composés.

10. Composition pharmaceutique anti-microbienne topique, selon l'une quelconque des revendications 7 à 9, dans laquelle le monoester est présent en une proportion de 0,025 à 20,0 % en poids par rapport au poids de la composition.

11. Composition pharmaceutique anti-microbienne topique, selon l'une quelconque des revendications 1 à 10, dans laquelle le support comprend un alcool, un agent chélateur, un tensio-actif, un parabène, de l'eau ou un mélange de ces composés.

12. Composition pharmaceutique anti-microbienne topique, selon la revendication 11, dans laquelle le support pharmaceutique comprend un alcool qui est le propylène-glycol, le phénoxyéthanol, le méthanol, l'éthanol, l'alcool isopropylique ou un mélange de ces composés, un agent chélateur qui est EDTA, $Na_2$(EDTA), $Na_4$(EDTA), TEA, l'acide lactique, l'acide citrique ou un mélange de ces composés, un tensio-actif qui est un sarcosinate, un oxyde d'amine, le pluronic 68, le laurylsulfate de sodium ou un mélange de ces composés, ou un parabène qui est le méthylparabène, le propylparabène ou un mélange de ces composés.

13. Composition pharmaceutique anti-microbienne topique, selon la revendication 11 ou 12, qui comprend 5 % à 60 % en volume de l'alcool, 0,25 % à 10 % en poids du tensio-actif, 0,05 % à 5 % en poids du parabène, et 0,05 % à 4 % en poids de l'agent chélateur.

16

**14.** Composition selon l'une quelconque des revendications 1 à 13, destinée à être utilisée comme agent anti-microbien topique.

**15.** Utilisation de la composition selon l'une quelconque des revendications 1 à 14 pour la préparation d'un médicament anti-microbien topique.

**Revendications pour l'Etat contractant suivant : ES**

**1.** Procédé de préparation d'une composition pharmaceutique anti-microbienne topique, qui comprend le mélange de :
(a) un ester d'acide gras du glycérol, éthoxylé ou propoxylé, et
(b) un support pharmaceutiquement acceptable

**2.** Procédé de préparation d'une composition pharmaceutique anti-microbienne topique, selon la revendication 1, qui comprend le mélange de :
(a) un ester d'acide gras du glycérol, éthoxylé ou propoxylé,
(b) un mélange d'acides gras comprenant :
(i) un premier acide gras, agent anti-microbien, qui est un acide gras en $C_6$ à $C_{18}$, et
(ii) un second acide gras, agent anti-microbien, qui est un acide gras en $C_6$ à $C_{18}$, le second acide gras étant différent du premier acide gras, et
(c) un support pharmaceutiquement acceptable.

**3.** Procédé selon la revendication 2, dans lequel le rapport en poids de l'ester au premier acide gras et au second acide gras combinés est de 1:10 à 10:1.

**4.** Procédé selon la revendication 2 ou 3, dans lequel le premier acide gras et/ou le second acide gras est un acide gras à chaîne droite, ayant de 6 à 12 atomes de carbone.

**5.** Procédé selon l'une quelconque des revendications 1 à 4, dans lequel l'ester du glycérol comprend au moins 1 mole de fragment éthoxy ou propoxy pour 4 moles d'ester de glycérol.

**6.** Procédé selon la revendication 5, dans lequel 0,5 à 5 moles de fragment: éthoxy ou propoxy sont présentes par mole d'ester de glycérol.

**7.** Procédé selon l'une quelconque des revendications 1 à 6, dans lequel l'ester d'acide gras du glycérol est un monoester.

**8.** Procédé selon la revendication 7, dans lequel le monoester contient 6 à 21 atomes de carbone.

**9.** Procédé selon la revendication 8, dans lequel le monoester est la monocapryline, la monocaprine, la monolaurine, la monomyristine, la monopalmitoléine, la monoléine, la monoicosénoïne, la monoérucine ou un mélange de ces composés.

**10.** Procédé selon l'une quelconque des revendications 7 à 9, dans lequel le monoester est présent en une proportion de 0,025 à 20,0 % en poids par rapport au poids de la composition.

**11.** Procédé selon l'une quelconque des revendications 1 à 10, dans lequel le support utilisé comprend un alcool, un agent chélateur, un tensio-actif, un parabène, de l'eau ou un mélange de ces composés.

**12.** Procédé selon la revendication 11, dans lequel le support pharmaceutique comprend un alcool qui est le propylène-glycol, le phénoxyéthanol, le méthanol, l'éthanol, l'alcool isopropylique ou un mélange de ces composés, un agent chélateur qui est EDTA, $Na_2$(EDTA), $Na_4$(EDTA), TEA, l'acide lactique, l'acide citrique ou un mélange de ces composés, un tensio-actif qui est un sarcosinate, un oxyde d'amine, le pluronic 68, le laurylsulfate de sodium ou un mélange de ces composés, ou un parabène qui est le méthylparabène, le propylparabène ou un mélange de ces composés.

**13.** Procédé selon la revendication 11 ou 12, dans lequel l'alcool est utilisé en une proportion de 5 % à 60 % en volume, le tensio-actif est utilisé en une proportion de 0,25 % à 10 % en poids, le parabène est utilisé en une proportion de 0,05 % à 5 % en poids, et l'agent chélateur est utilisé en une proportion de 0,05 % à 4 % en poids.

14. Composition telle que définie dans l'une quelconque des revendications 1 à 13, destinée à être utilisée comme agent anti-microbien topique.

15. Utilisation d'une composition telle que définie dans l'une quelconque des revendications 1 à 13 pour la préparation d'un médicament anti-microbien topique.